# EUROPEAN PATENT SPECIFICATION

(11) **EP 2 108 339 B1**
(45) Date of publication and mention of the grant of the patent: **04.07.2012**
(21) Application number: 07851847.9
(22) Date of filing: 04.12.2007
(51) Int. Cl.: A61F 2/42, A61F 2/58, A61F 2/68

(54) **FUNCTIONAL HAND PROSTHESIS MECHANISM**
FUNKTIONALER HANDPROTHESENMECHANISMUS
MÉCANISME DE PROTHÈSE FONCTIONNELLE POUR MAIN

(30) Priority: 17.01.2007 MX MX07000682
(43) Date of publication of application: 14.10.2009
(73) Proprietor: Bravo Castillo, Luis Armando, Estado de México (MX)
(72) Inventor: Bravo Castillo, Luis Armando, Estado de México (MX)
(74) Representative: Kaufmann, Ursula Josefine
(86) International application number: PCT/MX2007/000148
(87) International publication number: WO 2008/088204

(56) References cited:
- GB-A- 2 072 020
- US-A- 3 822 418
- US-A- 4 094 016
- US-A- 4 921 293
- US-A- 5 062 673
- US-A- 5 800 571
- US-A1- 2002 077 708
- US-B1- 7 087 092

## Description

### FIELD OF THE INVENTION

This present invention relates to a functional hand prosthesis mechanism according to the preamble of claim 1. Such a device is known from GB-A-2 072 020.

### BACKGROUND

For a long time the need for man to develop equipment or systems to help people who have suffered the loss of an upper or lower limb, either as a result of an accident or because of a birth defect, has brought engineering towards the creation of prostheses for the purpose of offering a different option to the amputees for their rehabilitation using technological developments that are being generated along these lines.

The first prostheses that were built were merely aesthetic, this means non-functional, later those emerged which were operated by means of mechanical systems, prostheses immediately appeared for the upper limb (arm) that replaced the traditional mechanical grip or hook prostheses, which were activated by means of a stub that followed the movements of the shoulder and already in the present time functional prostheses are coming out with greater technological developments that allow them to be more functional, ergonomic, easily operated and with a better aesthetic appearance, ever more approaching the appearance and working of a real leg or arm.

On the basis of the research that was carried out, we came across technologies which used different mechanisms focussed on the field of the prosthetic upper limb for humans, especially the hand, and those which have greater technical appearance to the functional prosthetic mechanism of a human hand are listed:
- Document US 5,013,326 describes an artificial human hand which is fitted with fingers turned at a specific curvature, the axis of these is kept at a 30° angle in respect of the axis of the forearm and which can open up to an angle of 70°. It also has a thumb, which has a sharp angle in respect of the plane of the forearm this allows it to take elements or lift small objects that are on a surface. The movement of the turned phalanges is transmitted by means of a half rack that goes inside of same.
- Document JP2080044 bases its working on a very similar mechanism to that described in the patent US 5013326, where the mechanical system corresponds to 3 hooks located in such a way that it simulates the thumb, index and middle fingers. It focuses part of its description on the actuator that generates the movement of the hand.
- Document WO0069375, 1968; deals with a prostheses of a hand which has an individual movement for each one of the 5 fingers, each finger has a separate actuator the control of which is made by means of strain gauges.
- Document US 5,200,679, 1993 describes a robotic hand that uses a prosthetic element which includes five fingers. It has a double turn motor which, with two cables generates the opening and closing of the hand. On turning the motor in one direction generates a tension on the first cable, which retracts the phalanges inwards towards the hand and generates the closing, subsequently the second cable generates a tension on the second cable, this by the inverse turn of the motor, it causes the fingers to return to their original extended position, this means, open hand.
- Document US 4,114,464 speaks of an artificial hand mechanism which is made up of at least one finger and the thumb and with these the opening and closing of same is generated by means of a meshing linked to each one of them, same which are engaged or adjusted to a worm mesh which is connected to a motor.
- Document US 4,377,305 describes the working of an artificial hand which has two holders that are jointed on a bolt which provides the opening and closing movement of the holders, in addition it provides the flexo-extension movement of same on the same quadrant or plane as the opening and closing, the holders can rotate as they are fixed on a parallel axis to the axis of the bolt. The opening and closing movement of the holders is made by means of a dual curved section rack and a straight mesh mechanically fixed to them. It also has the adduction-abduction movement and turning of the wrist.
- Document US 5,080,681, 1990 describes the working of an artificial hand that has two moveable phalanges and a hook that simulates the thumb, the opening and closing movement of the phalanges that is described in this prosthesis mechanism is made by means of a mobile device activated by means of an actuator, when the latter is activated, the mobile device slides along a guide that is in the chassis of the artificial hand. It has a system of artificial tendons which are connected to the mobile device on one side and at the rear of the phalanges, when the device is moved nearer (towards the patient's body) the tendons contract and generate the closure of the fingers and when the mobile is moved away, the tendons relax and this generates the opening of the fingers.

- Document US 5,888,246 describes the coupling between the worm and a mesh joined to a finger member: The worm transmits the movement to the mesh and the angular movement of a member that simulates a finger is generated, it has the option of being fitted to an artificial hand.
- Document US 5,378,033 describes a complex mechanism of a hand, which has three fingers fitted on a pair of plates that are toothed in three sections on their periphery, by means of which the turn of the hand is made by the meshes that transfer the movement coming from the motor. The opening and closing of the fingers is made by means of a mechanism that has a pair of linked conical gears, one is joined to the axis of the motor and the other to a cam with a follower, the latter is joined to three elements that are used as a guide at the base of the fingers, the maximum elevation path of the follower generates movement to the three guides connected to each finger, these transmit the movement to the base of the fingers that move on one of the discs, this generates a lever effect with which they open, this movement is transferred to the phalanges by means of a system of gears fitted from the base of the finger to the distal phalange on both sides with which the fingers go on opening, when the follower has its fall it generates the closure of the fingers.

- Document GB 2072020 describes the working of a prosthetic hand that uses a standard worm for the opening and losing action of the fingers, this is the one that is the closest to our invention. The fingers are supported by two parallel base plates which are attached to a wrist plate. An electrical actuator is provided for moving a nut on a worm from proximal to distal and vice versa, said worm being attached tc the shaft of the actuator at its proximal part. The nut is threaded on the worm and has a pair of projecting journals. These journals allow the nut to pivot within the yoke formed by two "thumb"-operating levers, and the end of the journals protrude beyond the levers to engage in slots in two "finger"-operating levers. The worm follows the arc path of the nut around a pivot mounting of the thumb member.
- Document CN 2680418Y describes the working of an artificial hand using a system of worm with the assistance of a lever or rod for the movement of the thumb.

Even though all of the above are functional in the field of the invention, there are some however that base their finger opening and closing working by means of a worm with a standard cord. In this present document the technical differences of our development are explained in regard to those already carried out, said differences are presented not only in the system of the moving of the fingers, but also in the plate that holders the elements, in the working of the fingers, etc. Preferred embodiments of the present invention is explained below in greater detail.

### DESCRIPTION OF THE FIGURES

Figure 1: Breakdown of the mechanism of the operational prosthesis for a hand.
Figure 2: Linkage of the elements described in Figure 1
Figure 3: Artificial wrist (16) and the elements that make it up
Figure 4: Finger assembly of the system
Figure 5: Detail of the index finger, the spring (2-a) and the tensioner (3-a) can be seen.
Figure 6: Base plate (5) of the hand elements
Figure 7: Longitudinal cross section of the base plate
Figure 8: 4 inlet worm (9-a) and bearing (14).
Figure 9: Movable unit (8)
Figure 10: A coupling for the fingers (2), (3) and (1) with worm (9) and movable unit (8).
Figure 11: Coupling of the movable unit (8) with worm (9).

### BRIEF DESCRIPTION OF THE INVENTION

The invention is defined in independent claim 1. Preferred embodiments are defined in dependent claims 2-9.

### DETAILED DESCRIPTION OF THE INVENTION

The functional hand prosthesis mechanism is made up of an artificial hand module with finger opening and closing movements, this action is generated by means of a worm system and a movable unit. The prosthesis is made by preference from aluminium, nylon, carbon fibre, stainless steel and brass. There is a detailed description below of each part of the systems in addition to its working and method of operation.

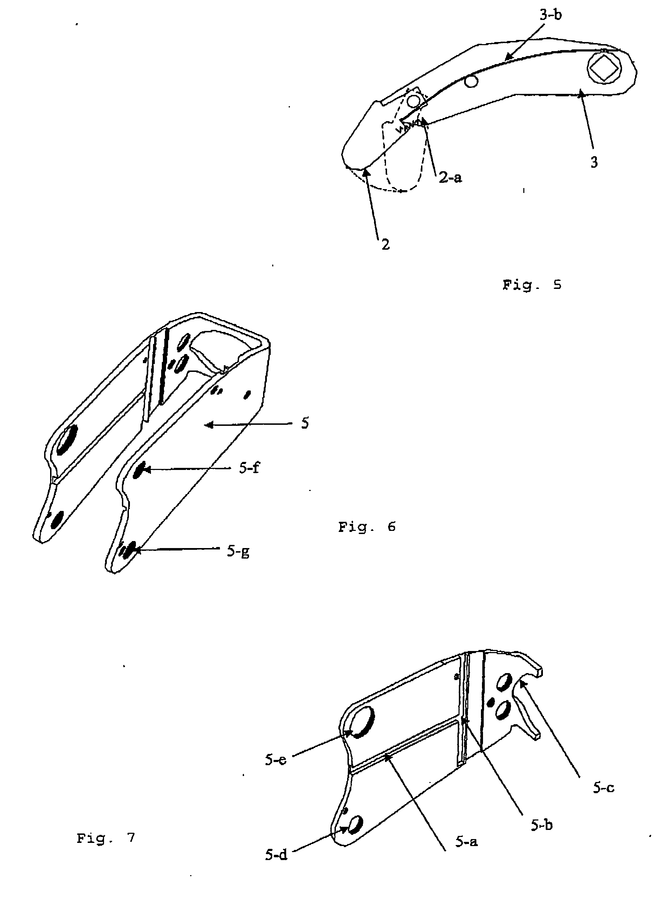

The functional hand prosthesis mechanism has a base plate (5) which is made from a light and highly resistant material, such as alumec 89 aluminium. Figure 6 shows the base plate (5) of the hand elements.

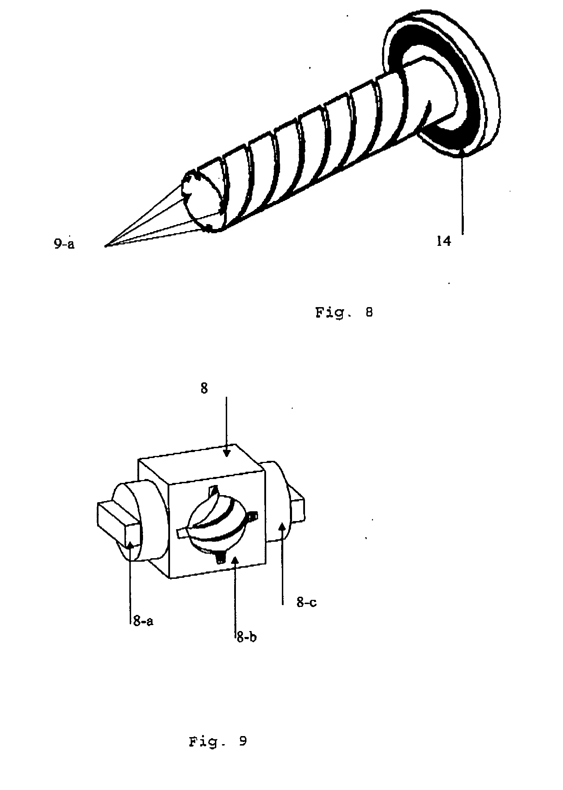

The peculiarity of this base plate is that in the machining on each of the inner walls there is a movement guide (5-a) from figure 7, parallel to axis of the actuator rotor (13) (in this case a direct current or DC motor is preferred) that is used so that the protrusions (8-a), figure 9 of the movable unit (8) slide in a lineal manner on the base plate (5) so as to generate the finger opening and closing action. There is also housing slot (5-b) of figure 7, machined in a vertical manner or perpendicular to the axis of the actuator rotor, which is used as housing for the security plate (10). This has the peculiarity of being used so as to avoid the shaft of the actuator from exiting the carcass of same when it has a very great pressure at the moment of lifting very heavy objects.

It also has an opening for the housing of the shafts on which the fingers are jointed and with an opening that houses the actuator (13), same is held or fixed to the base plate (5) by means of fixing elements, such as studs.

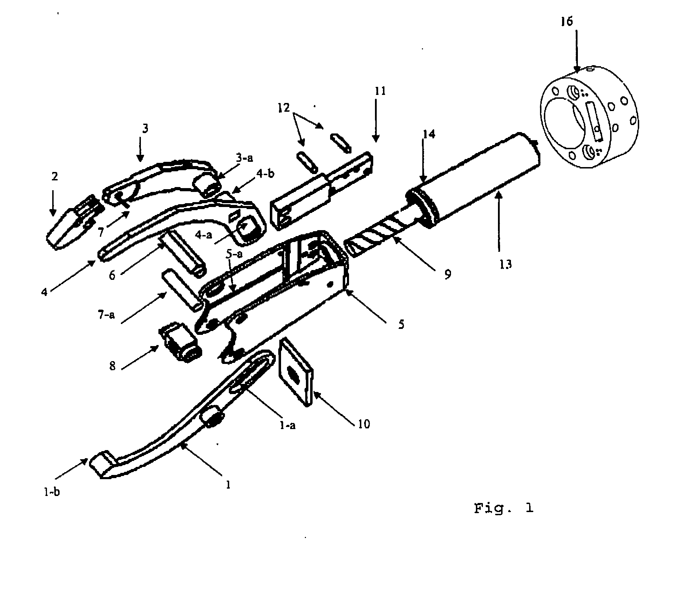

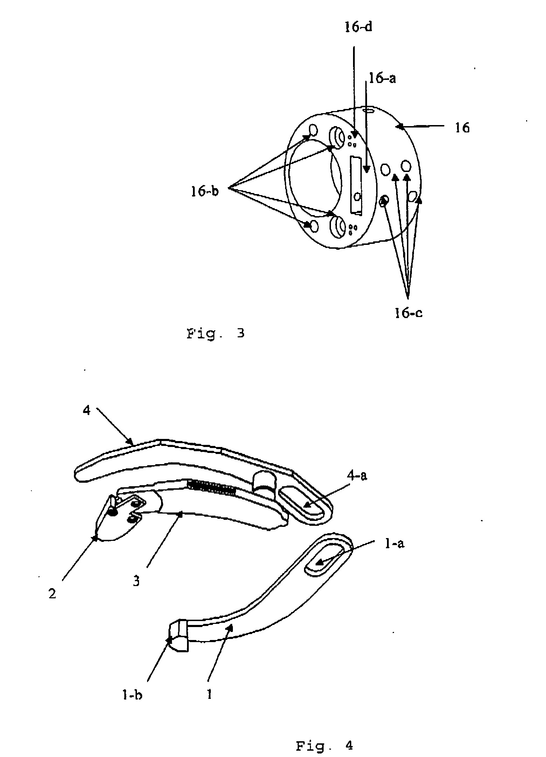

In order to give greater safety to the system there is an artificial wrist (16) figure 1, located at the external-proximal part of the base plate (5) which has a housing casing for the actuator (13) and a housing where one end of the linking plate (11) enters. Figure 3 shows the artificial wrist (16) and the elements that make it up.

The linking plate (11) figure 1 has the action of mechanically holding the artificial wrist with the linking plate by means of some fixing elements and hence providing base and connection to the actuator (13).

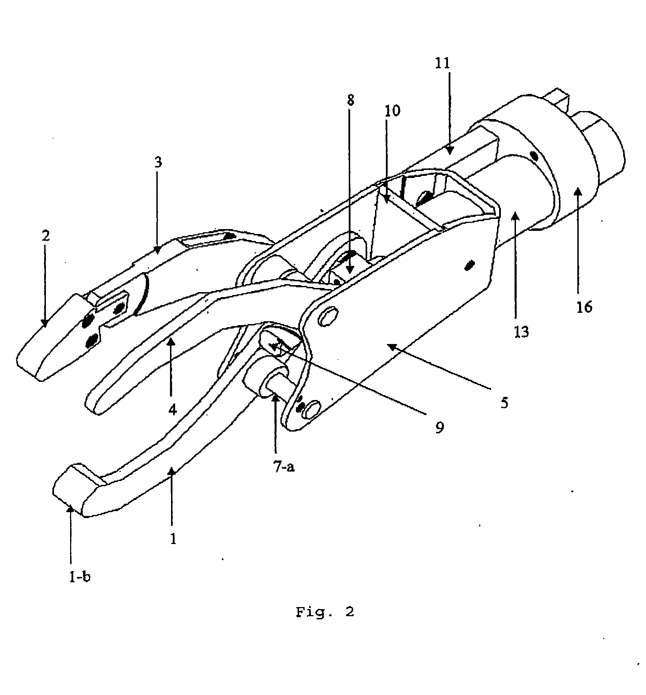

The artificial hand mechanism has only three fingers, as can be seen from figure 4 these are; thumb (1) with its tip (1-b) so as to make greater surface contact, index finger shaped by a jointed distal phalange (2) and by the index finger body (3) and finally by the middle finger (4); these elements are assembled onto a base plate (5) at its distal (front) part and are jointed on the base plate (5), it can be made by using studs (6) and (7-a). The jointed distal phalange (2), the index finger body (3), the middle finger (4) and the thumb (1) are assembled on the base plate (5) as shown in figure 2. Figure 5 depicts a detail of the index finger, the spring (2-a) and the tensioner (3-a) can be seen.

The element (2) joints on the index finger body (3) by means of the stud (7) of figure 1, in turn the protrusion (3-a) enters in the opening (5-e) located in the base plate (5) on the external part of (5), the middle finger (4) transfers the movement to (3) by means of the stud, in this case a stud with a rectangular shape (6) is used, which goes into the protrusion (3-a) and into protrusion (4-b) ; using figure 1 to support us we can see that the stud (6) has a cylindrical element at one of its ends, this is fitted into the opening (5-f) and is reinforced by means of a fixing element (such as a stud or fixer) to stop it coming out from the effect of the movement. Finally the thumb (1) is jointed by means of a stud (7-a) which is housed in the openings (5-d) and (5-g), the finger (1) is fixed in (7-a) by means of a fixing element.

The thumb (1) resists the other two and the opening and closing mechanism of same is carried out by means of the force created between the worm (9) and the moveable element or movable unit (8) and the movement is transferred by means of an actuator (13) by preference a DC motor).

The fingers (1) and (4) have a sliding guide, (1-a) and (4-a) respectively shown in figure 4, which are used to guide the protrusion (8-c) of the part (8), this allows an angular movement of the fingers by the effect of the lineal movement of the movable unit (8) of figure 9, which moves along the worm (9) that is mechanically joined to the actuator shaft (13).

In figure 9, we can see that the movable unit (8) has four inlets (8b) and with a pair of protrusions (8-a) that slide along the guides (5-a) machined in (5), which can be seen in greater detail in figure 7, this allows (8) to slide on (5) with greater ease keeping a completely lineal movement.

It is very important to mention that the worm (9) has a safety plate (10) that is housed in the base plate (5) which prevents (9) from sliding or having any play on (5), in addition it gives greater security to (13) when the patient lifts items with considerable weight; it must be highlighted that there is a guide (5-b) machined in (5) onto which the security plate is adjusted (10), these guides are used to limit the plate and with it prevent (10) from moving or having any type of movement on (5), a bearing (14) provides better adjustment and prevents friction between (9) and (10).

The speed with which the fingers open and close shown in figure 4, depend directly on the relationship that there is between the number of worm openings, in this case there are 4 openings (9-a of figure 8), and the diametric passage, however these parameters can vary depending on the needs that the system requires.

In the patent documents GB2072020 and CN2680418Y a worm is also used as a finger opening and closing activating system, these control or use a standard cord for the screw, in contrast to that which is used in the present invention. The problem that the previous documents present is that if at a specific moment the system for opening and closing the fingers needs a greater speed, this means, to open and close in less time, it requires a greater consumption of current from the actuator or actuators. Likewise the need to have greater speed, thus reduces the torque or power that there is in the DC motor, which has the effect that the fingers do not have sufficient strength so as to hold elements that are very heavy, on the other hand, if greater torque is needed, the speed at which the fingers open and close is considerably reduced.

Said problem is solved with the screw that is proposed in the present invention, as it is not standard due to the fact that it has 4 inlets instead of one and its advance passage is from between 4 and 5 times greater than the conventional ones. This allows it to have a considerable speed and high torque without having to provide it with greater power consumption. By using this type of 4 inlet worm (9) a good speed is achieved and with high torque, this means neither of the two parameters is sacrificed which is of absolute importance in the prosthetic hand and does not require greater power consumption.

In this present invention there is a relationship between the number of worm inlets with its advance passage, this determines the speed at which the movable unit moves on the worm, these parameters are adjusted depending on the needs of the patient.

The way in which this set of elements operates is as follows:
When the actuator (13) is energised with a positive-negative polarity, the shaft of same transfers its rotational movement to the worm (9), as they are mechanically linked, it is at this moment when, because of the same rotational action, the movable unit (8) that is coupled to (9) has lineal movement, parallel to the axis of the actuator rotor (13) from proximal to distal. This movement allows the fingers (1) and (4) to have an angular movement, thus generating the opening action of the fingers of the hand on engaging with the protrusion (8-c) of the movable unit (8) by means of the slide guides (1-a) and (4-a) respectively and articulated on the plate (5).

In so far as the closing of the fingers, the working is the same, the only thing that has to be done is to polarise the actuator in an inverse manner to the opening (negative-positive), thus generating a movement of the movable unit (8) from distal to proximal on the slide guides (5-a), in this way we have the closing action of the fingers on the hand.

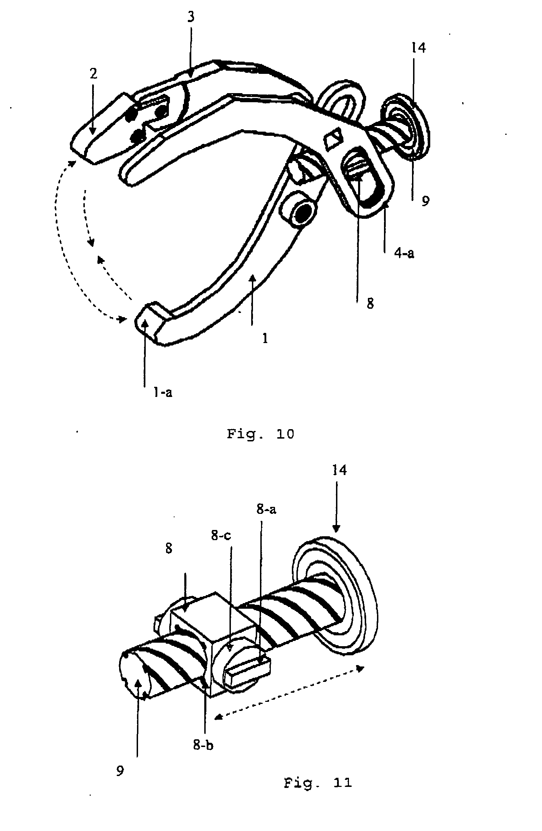

In figure 10 we can see the finger opening and closing movement, likewise the coupling on the movable unit (8) and the worm (9). Figure 11 illustrates the coupling of the movable unit (B) with worm (9).

It is very important to state that the system has electro-mechanical switches that prevent the finger opening and closing to go on operating at the maximum opening and closing limits, cutting the current that is sent to the actuator once the operating limit has been reached.

At the moment in which the closing of the fingers is carried out, the movement of the articulated phalange (2) on (3) is generated. This mechanism works in the following manner: The articulated phalange keeps an original position (extended) when the hand is open, this is achieved by means of a resistive or spring element (2-a) located between (2) and (3) as shown in figure 4. When the hand closes, there is an internal tensor element (3-a) in (3) that makes (2) retract inwards towards the hand, overcoming the force of the spring (2-a), this means compressing it in such a way that when the fingers reach their maximum closure, the tip (1-b) connects with the jointed phalange (2), this allows the patient to take small elements with greater precision and ease. When the hand opens, the spring (2-a) once again acts that is held under compression, making (2) return to its original position and the tensor (3-a) comes to rest. With this the artificial hand achieves a greater similarity to a biological hand, as normally the artificial hands that are available on the market have a thumb that is longer than a human thumb. The sizes of the functional hand prosthesis mechanism, in its mechanical elements, are subject in regard to the anatomy of the patient.

## Claims

1. A functional hand prosthesis mechanism, which includes gripping members (1; 2, 3; 4) supported by a base plate (5), an actuator (13) for moving a movable unit (8) on a worm (9) from proximal to distal and vice versa, said worm (9) being attached to the shaft of the actuator (13) at its proximal part, and a wrist part (16),
**characterized by**
- the base plate (5) formed in the shape of the letter "U" and having an opening in the central part (5-c) in which the actuator (13) is fitted;
- on the inner walls of the support plate (5)
- a pair of housing guides (5-b), where a safety plate (10) is housed, and
- a pair of movement guides (5-a) on which the movable unit (8) slide, said guides (5-a) allow a completely lineal movement of the movable unit (8) which is assembled onto a four inlet (9-a) worm (9) and it moves on this, from proximal to distal and vice versa;
- the gripping members (1; 2, 3; 4) being 3 fingers which are jointed and attached onto the distal end of the plate (5), the part furthest from the curvature of the support plate (5);
- the wrist part being an artificial wrist (16) located on the proximal rear part of the support plate (5) into which a part of the actuator body and a union plate (11) are housed.

2. The functional hand prosthesis mechanism according to claim 1, **characterised in that** the actuator (13) is a DC motor and the safety plate (10) work together with the wrist (16) so as to prevent the longitudinal movement of the actuator (13), the coupling plate (11) has an opening for the introduction of the studs (12) that fix the wrist (16) to this plate (11) by means of two openings (16-c); on the other end of these openings there are additional openings in the plate (11) so as to fix the coupling plate (11) to the base plate (5) so that the wrist (16) is fixed to the base plate (5).

3. The functional hand prosthesis mechanism according to claim 2, **characterised in that** the three fingers are:
a) the thumb (1), which has a tip (1-b) with a larger contact surface area for the elements to be picked up,
b) the index finger, made up of an articulated distal phalange (2) and an index finger body (3); and
c) the middle finger (4),where these elements are going to be assembled on the distal part of the support plate (5) and articulate on it around the studs (6 and 7-a), fitted in the openings (5d, 5e, 5f and 5g).

4. The functional hand prosthesis mechanism according to claim 3, **characterised in that** the index finger has a jointed distal phalange (2) that turns around a stud (7), this turn allows the closing of the fingers, the phalange against the tip (1-b) of the thumb (1), allowing the holding of small or slim elements; when the hand closes, the internal tensor (3-a) makes the jointed distal phalange (2) withdraw inwardly into the hand, modifying the state of the spring (2-a); when the hand opens, the elements work in reverse releasing the object that is held between the phalange (2) and the tip (1-b).

5. A functional hand prosthesis mechanism according to claim 4, **characterised in that** the thumb (1) and the middle finger (4) slide in a straight line on some movement guides (5-a) of the plate (5) by means of some extensions (8-a) of the protrusions (8-c) of the movable unit (8), the movement guides (4-a) and (1-a) are fitted in a manner that can turn on the protrusions (8-c), the longitudinal movement of the movable unit (8), from the turning effect of the screw (9) driving the opening and closing action of the fingers that turn on the actuator (13).

6. The functional hand prosthesis mechanism according to claim 5, **characterised in that** the openings (9-a) of the worm (9) connect with corresponding openings (8-b) of the movable unit (8), where the protrusions (8-a) that are fitted into the grooves (5-a) cause the movable unit (8) to slide longitudinally and not to turn when the worm (9) turns.

7. The functional hand prosthesis mechanism according to claim 6, **characterised in that** the artificial wrist has an opening to house the actuator and to hold it to the wrist by means of fixing elements through the openings (16-c), in addition it has a groove (15-a) of a size so as to fit with the connecting plate (11) by means of screws, in addition the wrist has additional openings for the fixing of a mechanical element or extension of the forearm.

8. The functional hand prosthesis mechanism according to claim 7, **characterised in that** the middle finger (4) and the index finger (3) move at the same time and in the same direction, the middle finger (4) transfers the movement to the index finger (3) by means of a stud (6), which goes into the protrusion (3-b) of the index finger and the protrusion (4-b) of the middle finger; the stud (6) has a cylindrical end so as to fit to the opening (5-f) and is fixed to the plate (5) by means of a fixing element such as a stud or fastener so as to prevent it from exiting from the effect of the movement.

9. The functional hand prosthesis mechanism according to claim 8, **characterised in that** the middle finger (4) is located in the plate (5), so that the index finger (3) is located on the outer part of the plate and where the thumb is located in the plate but on the opposing wall to the middle finger.

## Patentansprüche

1. Funktionaler Handprothesenmechanismus, umfassend von einer Trägerplatte (5) gehaltene Greifelemente (1; 2, 3; 4), einen Aktuator (13), der ein bewegliches Teil (8) auf einer Schraubspindel (9) von einer Nahstellung in eine entfernte Stellung und umgekehrt bringt, wobei besagte Schaubspindel (9) mit der Welle des Aktuators (13) in dessen Nahstellung verbunden ist, sowie ein Handgelenks-Teil (16),
**gekennzeichnet durch**
- die Trägerplatte (5), in Form des Buchstabens "U" geformt, und in ihrem Mittelteil (5-c) eine Öffnung aufweisend, in welche der Aktuator (13) eingepasst ist;
- auf der Innenseite der Seitenwände der Trägerplatte (5)
- ein Paar Halteführungen (5-b), in welchem eine Sicherungsplatte (10) untergebracht ist, und
- ein Paar Gleitführungen (5-a), in denen das bewegliche Teil (8) gleitet, wobei besagte Gleitführungen (5-a) eine vollständig geradlinige Bewegung des beweglichen Teils (8) ermöglichen, das seinerseits auf eine Schraubspindel (9) mit 4 Gängen (9-a) aufgesetzt ist und sich auf ihr von einer Nahstellung in eine entfernte Stellung und umgekehrt bewegt;
- die Greifelemente (1; 2, 3; 4), drei Finger bildend, welche gelenkig am entfernten Ende der Trägerplatte (5) angebracht sind, d.h. an dem von der Biegung der Trägerplatte (5) am weitesten entfernten Ende;
- das Handgelenks-Teil ,ein künstliches Handgelenk (16) bildend, welches sich an der nahen Rückseite der Trägerplatte (5) befindet, in welche ein Teil des Aktuator-Körpers und eine Verbindungsplatte (11) eingebaut sind.

2. Funktionaler Handprothesenmechanismus gemäß Anspruch 1, **dadurch gekennzeichnet, dass** der Aktuator (13) ein Gleichstrommotor ist und die Sicherungsplatte (10) mit dem Handgelenks-Teil (16) zusammenwirkt, um die Längsverschiebung des Aktuators (13) zu verhindern, dass die Verbindungsplatte (11) Öffnungen für das Einführen der Stifte (12) hat, mit denen das Handgelenks-Teil (16) an dieser Verbindungsplatte (11) mittels zweier Öffnungen (16-c) befestigt ist, und dass sich am diesen Öffnungen gegenüberliegenden Ende der Verbindungsplatte (11) zusätzliche Öffnungen in der Verbindungsplatte (11) befinden, mittels derer sich die Verbindungsplatte (11) mit der Trägerplatte (5) so verbinden lässt, dass das Handgelenks-Teil (16) an der Trägerplatte (5) befestigt ist.

3. Funktionaler Handprothesenmechanismus gemäß Anspruch 2, **dadurch gekennzeichnet, dass** die drei Finger sind:
a) der Daumen (1) mit der Spitze (1-b) mit einer größeren Kontaktfläche für die zu ergreifenden Teile,
b) der Zeigefinger, der aus einem beweglichen Fingerglied (2) am vorderen Ende und einem Zeigefinger-Körper (3) besteht, und
c) der Mittelfinger (4),
wobei diese Teile am entfernten Ende der Trägerplatte (5) montiert und mittels der in die Öffnungen (5-d, 5-e, 5-f und 5-g) eingesetzten Stifte (6 und 7-a) gelenkig mit ihr verbunden sind.

4. Funktionaler Handprothesenmechanismus gemäß Anspruch 3, **dadurch gekennzeichnet, dass** der Zeigefinger ein am vorderen Ende angebrachtes Fingerglied (2) besitzt, das sich um einen Stift (7) dreht, wobei diese Drehung das Schließen der Finger ermöglicht, wobei das Fingerglied gegen die Spitze (1-b) des Daumens (1) drückt, so dass kleine oder schmale Teile gehalten werden können; dass beim Schließen der Hand ein inneres Zugelement (3-a) das vorne angebrachte Fingerglied (2) nach innen in Richtung zur Hand schwenkt, wobei es den Zustand der Feder (2-a) verändert; und dass beim Öffnen der Hand diese Elemente umgekehrt wirken und dabei das zwischen dem Fingerglied (2) und der Spitze (1-b) gehaltene Teil wieder loslassen.

5. Funktionaler Handprothesenmechanismus gemäß Anspruch 4, **dadurch gekennzeichnet, dass** der Daumen (1) und der Mittelfinger (4) mittels einiger Erweiterungen (8-a) an den Vorsprüngen (8-c) des beweglichen Teils (8) in den Gleitführungen (5-a) der Trägerplatte geradlinig gleiten, wobei die Führungen (4-a) und (1-a) so ausgebildet sind, dass sie sich auf den Vorsprüngen (8-c) drehen können, während die durch die Drehung der Schraubspindel (9) verursachte Längsbewegung des beweglichen Teils (8) ein Öffnen oder Schließen der Finger bewirkt, sobald sich der Aktuator (13) dreht.

6. Funktionaler Handprothesenmechanismus gemäß Anspruch 5, **dadurch gekennzeichnet, dass** die Vertiefungen (9-a) in der Schraubspindel (9) mit den entsprechenden Vertiefungen (8-b) im beweglichen Teil (8) in Verbindung stehen, wobei die in die Laufrillen (5-a) eingreifenden Erweiterungen (8-a) dafür sorgen, dass sich das bewegliche Teil (8) geradlinig längs bewegt und sich nicht dreht, wenn sich die Schraubspindel (9) dreht.

7. Funktionaler Handprothesenmechanismus gemäß Anspruch 6, **dadurch gekennzeichnet, dass** das künstliche Handgelenk eine Öffnung für den Einbau des Aktuators besitzt und dieser Aktuator mittels in die Öffnungen (16-c) eingesetzten Halteelementen am Handgelenk befestigt ist; zusätzlich hat es eine Nut (15-a), deren Größe an die Verbindungsplatte (11) angepasst ist, um diese darin mittels Schrauben zu befestigen; weiterhin hat das Handgelenk zusätzliche Öffnungen zur Befestigung eines mechanischen Elements oder einer Verlängerung des Unterarms.

8. Funktionaler Handprothesenmechanismus gemäß Anspruch 7, **dadurch gekennzeichnet, dass** sich der Mittelfinger (4) und der Zeigefinger (3) gleichzeitig und in derselben Richtung bewegen, wobei der Mittelfinger (4) die Bewegung auf den Zeigefinger (2) mittels eines Zapfens (6) überträgt, welcher in den Vorsprung (3-b) des Zeigefingers und den Vorsprung (4-b) des Mittelfingers eingreift, und dass der Zapfen (6) ein zylindrisches Ende hat, mit dem er in Öffnung (5-f) passt und seinerseits mittels eines Befestigungselements wie einem Zapfen oder einem Halter an der Trägerplatte (5) befestigt ist, so dass er bei einer Bewegung nicht herausfällt.

9. Funktionaler Handprothesenmechanismus gemäß Anspruch 8, **dadurch gekennzeichnet, dass** der Mittelfinger (4) in der Trägerplatte (5) so angeordnet ist, dass der Zeigefinger (3) am äußeren Teil der Trägerplatte angeordnet ist, und dass der Daumen innerhalb der Trägerplatte, jedoch auf der dem Mittelfinger gegenüberliegenden Seitenwand angeordnet ist.

## Revendications

1. Mécanisme de prothèse fonctionnelle de la main, comprenant des éléments de préhension (1 ; 2, 3 ; 4) maintenus par une plaque de base (5), un actionneur (13) pour déplacer une unité mobile (8) sur une vis sans fin (9) d'une position proximale à une position distale et vice-versa, ladite vis sans fin (9) étant reliée à l'axe de l'actionneur (13) sur sa partie proximale, et une partie servant de poignet (16),
**caractérisé par**
- la plaque de base (5) en forme de U présentant dans sa partie centrale (5-c) une ouverture dans laquelle est logée l'actionneur (13) ;
- sur la face intérieure de la plaque de base (5)
- une paire de rails de maintien (5-b), dans laquelle est logée une plaque de sécurité (10), et
- une paire de rails de mouvement (5-a) dans laquelle se déplace l'unité mobile (8), sachant que lesdits rails (5-a) permettent un mouvement complètement linéaire de l'unité mobile (8) qui est disposée sur une vis sans fin (9) à quatre filets (9) et qui se déplace sur celle-ci, de la position proximale à la position distale et vice-versa ;
- les éléments de préhension (1 ; 2, 3 ; 4) sont 3 doigts articulés et reliés à l'extrémité distale de la plaque (5), c'est-à-dire la partie la plus éloignée de la courbe de la plaque de base (5) ;
- la partie servant de poignet est un poignet artificiel (16) situé sur la partie arrière proximale de la plaque de base (5) dans laquelle sont logées une partie du corps de l'actionneur et une plaque de liaison (11).

2. Mécanisme de prothèse fonctionnelle de la main selon la revendication 1, **caractérisé en ce que** l'actionneur (13) est un moteur à courant continu et que la plaque de sécurité (10) fonctionne en interaction avec le poignet (16) de manière à empêcher le mouvement longitudinal de l'actionneur (13), la plaque de liaison (11) présentant une ouverture pour l'introduction des goujons (12) qui fixent le poignet (16) à cette plaque (11) au moyen de deux ouvertures (16-c) ; et que à l'extrémité opposée à ces ouvertures se trouvent d'autres ouvertures dans la plaque (11) de manière à fixer la plaque de liaison (11) à la plaque de base (5) de sorte que le poignet (16) est fixé à la plaque de base (5).

3. Mécanisme de prothèse fonctionnelle de la main selon la revendication 2, **caractérisé en ce que** les trois doigts sont :
a) le pouce (1) qui a une pointe (1-b) présentant une grande surface de contact pour les éléments à saisir,
b) l'index composé d'une phalange distale articulée (2) et d'un corps d'index (3) ; et
c) le majeur (4),
ces éléments étant montés sur la partie distale de la plaque de base (5) et articulés sur celle-ci à l'aide des goujons (6 et 7-a) logés dans les ouvertures (5d, 5e, 5f et 5g).

4. Mécanisme de prothèse fonctionnelle de la main selon la revendication 3, **caractérisé en ce que** l'index présente une phalange (2) articulée à l'extrémité distale qui tourne autour d'un goujon (7), laquelle rotation permet la fermeture de la main, avec la phalange contre la pointe (1-b) du pouce (1), permettant la préhension d'éléments de petite taille ou de faible épaisseur ; que, lorsque la main se ferme, un tenseur interne (3-a) entraîne le repli de la phalange (2) assemblée à l'extrémité distale vers l'intérieur de la main, modifiant ainsi l'état du ressort (2-a) ; que, lorsque la main s'ouvre, ces éléments ont une action inverse, libérant ainsi l'objet tenu entre la phalange (2) et la pointe (1-b).

5. Mécanisme de prothèse fonctionnelle de la main selon la revendication 4, **caractérisé en ce que** le pouce (1) et le majeur (4) se déplacent en ligne droite dans les rails de mouvement (5-a) de la plaque (5) au moyen d'extensions (8-a) des saillies (8-c) de l'unité mobile (8), sachant que les ouvertures de guidage (4-a) et (1-a) sont conçus de telle manière qu'ils peuvent tourner sur les saillies (8-c), et que le mouvement longitudinal de l'unité mobile (8) entraîné par la rotation de la vis sans fin (9) entraîne l'ouverture et la fermeture de la main avec l'actionneur (13) en marche.

6. Mécanisme de prothèse fonctionnelle de la main selon la revendication 5, **caractérisé en ce que** les ouvertures (9-a) de la vis sans fin (9) sont reliées à des ouvertures correspondantes (8-b) de l'unité mobile (8), sachant que les saillies (8-a) logées dans les rails (5-a) entraînent le déplacement longitudinal de l'unité mobile (8) et l'empêchent de tourner lorsque la vis sans fin (9) tourne.

7. Mécanisme de prothèse fonctionnelle de la main selon la revendication 6, **caractérisé en ce que** le poignet artificiel présente une ouverture pour loger l'actionneur et pour le tenir contre le poignet au moyen d'éléments de fixation à travers les ouvertures (16-c), qu'il présente en outre une rainure (15-a), dont la taille est adaptée à la plaque de liaison (11) et dans laquelle celle-ci est fixée au moyen de vis, et que le poignet présente en plus des ouvertures pour fixer un élément mécanique ou un prolongement de l'avant-bras.

8. Mécanisme de prothèse fonctionnelle de la main selon la revendication 7, **caractérisé en ce que** le majeur (4) et l'index (3) se déplacent au même moment et dans la même direction, que le majeur (4) transfère le mouvement à l'index (3) au moyen d'un goujon (6) qui se prolonge dans la saillie (3-b) de l'index et la saillie (4-b) du majeur ; que le goujon (6) présente une extrémité cylindrique adaptée à l'ouverture (5-f) et est fixé à la plaque (5) au moyen d'un élément de fixation tel qu'un goujon ou une attache de manière à ne pas pouvoir se détacher sous l'effet du mouvement.

9. Mécanisme de prothèse fonctionnelle de la main selon la revendication 8, **caractérisé en ce que** le majeur (4) est disposé de telle manière dans la plaque (5) que l'index (3) est situé à l'extrémité extérieure de la plaque, et que le pouce est disposé dans la plaque, mais sur la paroi opposée au majeur.
